(19) Europäisches Patentamt
European Patent Office
Office européen des brevets

(11) EP 0 721 946 A1

(12) **EUROPÄISCHE PATENTANMELDUNG**

(43) Veröffentlichungstag: **17.07.1996 Patentblatt 1996/29**

(51) Int. Cl.[6]: **C07D 277/80**

(21) Anmeldenummer: **95120539.2**

(22) Anmeldetag: **27.12.1995**

(84) Benannte Vertragsstaaten: **BE DE ES FR GB IT NL**

(30) Priorität: **13.01.1995 DE 19500794**

(71) Anmelder: **BAYER AG**
**51368 Leverkusen (DE)**

(72) Erfinder:
- **Arend, Günther, Dr.**
  **D-41540 Dormagen (DE)**
- **Sicheneder, Adolf, Dr.**
  **D-41540 Dormagen (DE)**
- **Nehoda, Wilhelm, Dr.**
  **B-2930 Braschaat (BE)**

(54) **Verfahren zur Herstellung von N,N-Dialkyl-, -Dicycloalkyl, -Diaryl- oder -Diaralkyl-benzthiazolyl-sulfenamiden**

(57) N,N-Dialkyl-, -Dicycloalkyl-, -Diaryl- oder -Diaralkyl-benzthiazolyl-sulfenamide, die gegebenenfalls substituiert sein können, werden hergestellt, indem man gegebenenfalls substituierte 2-Mercaptobenzthiazole und/oder entsprechende Disulfide mit N,N-Dialkyl-, -Dicycloalkyl-, -Diaryl- oder -Diarylalkylaminen bei erhöhter Temperatur umsetzt, wobei man die Umsetzung in Gegenwart von Chlor oder in Gegenwart eines Chlor-Inertgasgemisches sowie in Gegenwart von wasserfreien $C_1$-$C_4$-Alkoholen durchführt und wobei die Chlorierungszeit 10 bis 150 Minuten, das Molverhältnis von gegebenenfalls substituiertem 2-Mercapto-benzthiazol zu Amin 1:3 bis 1:4 und das Molverhältnis von gegebenenfalls substituiertem Dibenzthiazolyldisulfid zu Amin 1:4 bis 1:6 beträgt und wobei 300 bis 1500 g Alkohol pro Äquivalent 2-Mercaptobenzthiazol und 1 bis 1,6 Mol Chlor pro Mol gegebenenfalls substituiertem 2-Mercapto-benzthiazol oder 1 bis 1,5 Mol Chlor pro Mol gegebenenfalls substituiertem Dibenzthiazolyldisulfid zugesetzt werden.

EP 0 721 946 A1

## Beschreibung

Die Erfindung betrifft ein Verfahren zur Herstellung von N,N-Dialkyl-, -Dicycloalkyl, -Diaryl- oder Diaralkyl-benzthiazolyl-sulfenamiden aus gegebenenfalls substituierten 2-Mercapto-benzthiazolen oder den entsprechenden Disulfiden und N,N-Dialkyl-, -Dicycloalkyl-, -Diaryl- oder -Diaralkyl-aminen durch Oxidation mit Chlor. Die so erhaltenen Produkte werden vor allem als langsam wirkende Vulkanisationsbeschleuniger in der Kautschukindustrie eingesetzt.

So ist z.B. N,N-Dicyclohexyl-benzthiazolyl-sulfenamid (DCBS) seit langem bekannt und durch verschiedene Verfahrensweisen zugänglich. Eines der technisch bedeutsamsten Verfahren wird beispielsweise in DE 32 33 395 beschrieben. Ausgehend vom Natriumsalz des 2-Mercaptobenzthiazols (NaMBT) und Dicyclohexylamin erhält man in einem Gemisch aus i-Propanol und Wasser das Produkt durch Oxidation mit Chlorbleichlauge. Dabei wird durch Zugabe von Säure ein konstanter pH-Wert eingestellt. Da das Thiazol im Überschuß eingesetzt wird, erhält man große Mengen an Benzthiazol-2-sulfonsäure. Zudem fallen große Mengen an Salz an, so daß das Verfahren als ökologisch bedenklich angesehen werden muß. Das in EP 314 663 beschriebene Verfahren weicht von dem in DE 32 33 395 dargestellten Verfahren nur marginal ab. Neben mäßigen Ausbeuten und verbesserungsbedürftiger Qualität hat man demzufolge auch ökologische Probleme zu berücksichtigen.

Eine Verschlechterung im Hinblick auf Ausbeute und Qualität stellt das in US 2 930 794 beschriebene Verfahren dar. Hierbei wird in Aceton gelöstes N-Chlor-dicyclohexylamin mit einer wäßrigen NaMBT-Lösung umgesetzt. Produkt von guter Qualität erhält man aber erst nach einem mit Produktverlust verbundenen Umkristallisationsschritt.

Ausbeuten im Bereich von lediglich 75 % sind mit dem in GB 835 782 beschriebenen Verfahren erhältlich. Das Verfahren basiert auf der Kondensation von Dicyclohexylamin mit Benzthiazolyl-2-sulfenylchlorid. Dessen Darstellung und Handhabung gestaltet sich allerdings schwierig im industriellen Maßstab.

Elektrolytische Herstellverfahren, wie sie beispielsweise in JP 76/118 981 beschrieben werden, sind in DMF unter Zusatz von quartären Ammoniumsalzen der Perchlorsäure durchzuführen. Eine großtechnische Anwendung ist sicherlich problematisch und unter Umweltaspekten als fragwürdig anzusehen.

In der Patentschrift EP 0 131 776, welche ein Verfahren unter Verwendung von Sauerstoff als Oxidationsmittel beschreibt, wird auch die zu DCBS führende Variante beansprucht. Diese Ergebnisse konnten aber in eigenen Versuchen nicht bestätigt werden und darüber hinaus ist dieser Prozeß sicherheitstechnisch bedenklich.

Eine andere Variante wird in JP 59/172 483 beschrieben. Hierbei erhält man das gewünschte Produkt durch Umsetzung von N- Chlor-dicyclohexylamin mit MBT oder MBTS in Gegenwart von Alkalihydroxiden oder Alkalialkoxiden (z.B. Namethanolat). Reste von Wasser sind bei diesem vorzugsweise in Methanol durchgeführten Verfahren überaus störend. Aus diesem Grund können Alkalihydroxide nur in alkoholischer Lösung eingesetzt werden. Auch die Abtrennung von Wasser nach der Herstellung von N-Chlor-dicyclohexylamin muß mit großer Sorgfalt erfolgen. Außerdem ist die Handhabung von N-Chlordicyclohexylamin aus sicherheitstechnischen Gründen nicht unproblematisch.

Aufgabe der vorliegenden Erfindung war es nun, ein wirtschaftliches Verfahren zur Herstellung von N,N-substituierten Benzthiazolsulfenamiden, insbesondere von N,N-Dicyclohexyl-benzthiazolyl-sulfenamid, in hoher Ausbeute bei hoher Selektivität und sehr guter Qualität, ausgehend von substituierten oder unsubstituierten 2-Mercaptobenzthiazolen oder deren Disulfide unter Berücksichtigung sicherheitstechnischer Aspekte, vorzugsweise in einer Eintopfreaktion, zur Verfügung zu stellen.

Gegenstand der vorliegenden Erfindung ist daher ein Verfahren zur Herstellung von N,N-Dialkyl-, -Dicycloalkyl-, -Diaryl- oder -Diaralkyl-benzthiazolylsulfenamiden durch Umsetzung von gegebenenfalls substituierten 2-Mercaptobenzthiazolen und/oder den entsprechenden Disulfiden mit N,N-Dialkyl-, -Dicycloalkyl, -Diaryl- oder -Diarylalkylaminen bei erhöhter Temperatur, das dadurch gekennzeichnet ist, daß man die Umsetzung in Gegenwart von Chlor oder in Gegenwart eines Chlor/Inertgasgemisches sowie in Anwesenheit von wasserfreien $C_1$-$C_4$-Alkoholen durchführt, wobei die Chlorierungszeit 10 bis 150 Minuten und das Molverhältnis von gegebenenfalls substituierten 2-Mercapto-benzthiazol zu dem entsprechenden Amin 1:3 bis 1:4,5 sowie das Molverhältnis von gegebenenfalls substituiertem Dibenzthiazolyldisulfid zu dem entsprechenden Amin 1:4 bis 1:6 beträgt und wobei 300 bis 1500 g Alkohol pro Äquivalent 2-Mercaptobenzthiazol und 1.0 bis 1,6 Mol Chlor pro Mol 2-Mercapto-benzthiazol oder 1,0 bis 1,5 Mol Chlor pro Mol Dibenzthiazolyldisulfid eingesetzt werden.

Das erfindungsgemäße Verfahren läßt sich durch folgende Reaktionsgleichungen ausgehend von MBTS verdeutlichen:

$$\text{BT-S-S-BT} + 4\text{HNR}_2 + \text{Cl}_2 \xrightarrow{\text{Alkohol}} 2\text{BT-S-NR}_2 + 2\text{R}_2\text{NH}_2^+\text{Cl}^- \quad (1)$$

$$2R_2NH_2^+Cl^- + 2NaOH + Cl_2 \longrightarrow 2HNR_2 + 2NaCl + 2H_2O \qquad (2)$$

---

$$BT\text{-}S\text{-}S\text{-}BT + 2HNR_2 + 2NaOH + Cl_2 \xrightarrow{\text{Alkohol}} 2BT\text{-}S\text{-}NR_2 + 2NaCl + 2H_2O$$

BT = Benzthiazolyl (auch substituiert); R = alkyl-, cycloalkyl-, aryl-, aralkyl

Die eingesetzten 2-Mercapto-benzthiazole können Chlor, $C_1$-$C_6$-Alkyl-, $C_1$-$C_6$-Alkoxy- oder Nitrogruppen als Substituenten am Benzolring tragen. Bevorzugt aber wird 2-Mercaptobenzthiazol eingesetzt.

Selbstverständlich ist es auch möglich, wie erwähnt, die entsprechenden Disulfide der genannten Mercaptobenzthiazole einzusetzen.

Als N,N-Dialkyl-, -Dicycloalkyl-, -Diaryl- oder -Diarylalkyl-amine kommen für das erfindungsgemäße Verfahren in Frage Dipropylamine, Dibutylamine, Dipentylamine, Dihexylamine, Dicyclopentylamin, Dicyclohexylamin, Dicycloheptylamin, Cyclohexyl-cyclopentylamin, Di-(methyl-cyclohexyl)-amine, Di-(dimethyl-cyclohexyl)-amine, Di-(trimethylcyclohexyl)-amine, Anilin, N-Methyl-anilin, Dibenzylamin, Benzyl-methylamin, bevorzugt Diisopropylamin, Dibenzylamin, Dicyclohexylamin.

Das erfindungsgemäße Verfahren wird bevorzugt bei Temperaturen von 40 bis 80°C, ganz besonders bevorzugt 55 bis 65°C durchgeführt.

Wie erwähnt, wird die erfindungsgemäße Umsetzung in Gegenwart von Chlor oder in Gegenwart eines Chlor-Stickstoffgemisches durchgeführt. Das Inertgas kann dem Chlor in einer Menge von 10 bis 90 Gew.-%, bevorzugt 40 bis 60 Gew.-% zugegeben werden.

Das erfindungsgemäße Verfahren wird in Gegenwart von $C_1$-$C_4$-Alkoholen, wie Methanol, Ethanol, Isopropanol, n-Butanol, tert.-Butanol oder Gemischen dieser Alkohole durchgeführt. Dabei ist es von Bedeutung, daß die Alkohole in wasserfreier Form (< 1 Gew.-% Wasser) eingesetzt werden.

Da die Löslichkeit der erhaltenen Sulfenamide in der Regel mit steigendem Molekulargewicht des Alkohols zunimmt, wird die Reaktion vorzugsweise in Methanol durchgeführt. Von Vorteil ist dabei auch, daß Methanol kein Azeotrop mit Wasser bildet (wichtig für die Lösungsmittelrückgewinnung) und bei einem Siedepunkt von 64,6°C gut für ein Arbeiten unter Siedekühlung geeignet ist.

Bei dem erfindungsgemäßen Verfahren werden bevorzugt 300 bis 1300 g Alkohol pro Äquivalent 2-Mercaptobenzthiazol eingesetzt.

Außerdem werden bevorzugt 1 bis 1,4 Mol Chlor pro Mol Mercaptobenzthiazol oder 1 bis 1,25 Mol Chlor pro Mol Dibenzthiazolyldisulfid in das erfindungsgemäße Verfahren eingesetzt.

Nach dem erfindungsgemäßen Verfahren beträgt das Molverhältnis von 2-Mercapto-benzthiazol zu den genannten Aminen bevorzugt 1:3 bis 1:4 und das Molverhältnis von den entsprechenden Dibenzthiazolyldisulfiden zu den Aminen bevorzugt 1:4,3 bis 1:5.

Für das erfindungsgemäße Verfahren ist es wesentlich, daß die Chlorierungszeit sich in einem bestimmten Rahmen bewegt. Bevorzugt beträgt die Chlorierungszeit 15 bis 60 Minuten, ganz besonders bevorzugt 15 bis 30 Minuten. Ist die Chlorierungzeit zu lang oder zu kurz, so kann dies Auswirkungen auf die Ausbeute und die Selektivität der Reaktion haben. Die günstigste Chlorierungszeit kann leicht durch Vorversuche ermittelt werden.

Die Zugabe des Chlors bzw. des Chlor/Inertgasgemisches wird üblicherweise so durchgeführt, daß man über eine Düse bzw. ein Kapillarrohr, das in die Reaktionsmischung eingetaucht ist, unter Rühren das Gas einleitet.

Darüber hinaus ist es in Bezug auf Ausbeute und Selektivität der Reaktion von Bedeutung, daß ein bestimmtes Verhältnis, wie oben beschrieben, von Amin zu Mercaptobenzthiazol oder dem entsprechenden Disulfid eingehalten wird. Wird eine zu große Menge an Aminen zugegeben, so verbessert dies nicht die Selektivität der Reaktion, sondern erhöht die Löslichkeit des Produktes in der Mutterlauge und führt somit zu Ausbeuteverlusten.

Das erfindungsgemäße Verfahren kann sowohl kontinuierlich als auch diskontinuierlich durchgeführt werden. Bevorzugt wird das erfindungsgemäße Verfahren in diskontinuierlicher Fahrweise in einer sogenannten Eintopfreaktion durchgeführt.

Im Falle der Herstellung von DCBS verfährt man im allgemeinen so, daß man 2-Mercaptobenzthiazol bzw. Benzthiazolyldisulfid und Dicyclohexylamin im gewünschten Alkohol löst, oder suspendiert, und auf Reaktionstemperatur, beispielsweise 60°C (im Falle von Methanol empfiehlt sich eine Erwärmung auf Siedetemperatur), aufheizt. Unter Rühren leitet man dann die benötigte Menge an Chlor über eine Düse bzw. ein Kapillarrohr, das in die Reaktionsmischung

eingetaucht ist, ein. Im Hinblick auf gute Ausbeuten sollte die Einleitdauer so kurz wie möglich, beispielsweise 30 Minuten, gewählt werden Nach einer Nachrührzeit von etwa 1,5 Stunden gibt man wäßrige Alkalihydroxidlösung zu der Reaktionsmischung um das Amin wieder freizusetzen. Das gewünschte Reaktionsprodukt liegt dann als Feststoff suspendiert in der Mutterlauge vor und kann durch Filtration abgetrennt werden.

Nach dem erfindungsgemäßen Verfahren erhält man die gewünschten Produkte in einer hohen Reinheit (>99 %). Der Schmelzpunkt der Reaktionsprodukte liegt in der Regel über 102°C. Da das erfindungsgemäße Verfahren mit hoher Selektivität abläuft, ist auch die Nebenproduktbildung sehr gering.

**Beispiele**

Die vorliegende Erfindung wird durch die nachfolgenden Versuchsbeispiele näher erläutert:

**Beispiel 1**

In einem 3 l-Planschliffbecher mit Rührer, Rückflußkühler, Thermometer, Tropftrichter und Gaseinleitungsrohr (Kappilare 1 mm) wurden 250 g (0,752 Mol) Dibenzthiazolyldisulfid, 640 g (3,53 Mol) Dicyclohexylamin und 1800 g Methanol (Wassergehalt <1 %) vorgelegt und auf 60°C erwärmt. Dann leitete man innerhalb von 30 Minuten 64 g (0,9 Mol) Chlor über das Kapillarrohr ein. Anschließend rührte man die Reaktionsmischung 90 Minuten bei 60°C nach.

Innerhalb von 15 Minuten gab man dann 315 g (1,97 Mol) wäßrige, 25 %ige Natronlauge zu und rührte weitere 30 Minuten nach. Schon während der Nachrührzeit begann man mit der Kühlung der Rektionsmischung auf schließlich 20°C. Das Produkt wurde abfiltriert, mit 500 g Methanol und anschließend 2 x 500 g Wasser gewaschen und im Trokkenschrank (50°C, 200 mbar) getrocknet.

Man erhielt 476 g (1,373 Mol) Dicyclohexyl-benzthiazolyl-sulfenamid mit einem Schmelzpunkt von 103°C. Laut HPLC-Analyse liegt die Reinheit bei über 99 %. Die Ausbeute an DCBS betrug demnach 91,3 % der Theorie, bezogen auf eingesetztes MBTS.

In der Mutterlauge blieben 21 g DCBS gelöst. Demnach lag die Selektivität der Reaktion bei 95 %.

**Beispiel 2**

Es wurde wie in Beispiel 1 gearbeitet, aber mit einer Chlorierungszeit von 60 Minuten. Die Ausbeute an DCBS betrug 445 g (85,4 %), bei einer Selektivität von 91 %.

**Beispiel 3**

Es wurde wie in Beispiel 1 gearbeitet, aber mit einer Chlorierungszeit von 120 Minuten. Die Ausbeute an DCBS betrug 418 g (80 %), bei einer Selektivität von 86 %.

**Beispiel 4**

Die Menge an eingesetztem Lösungsmittel (Methanol) wurde gegenüber Beispiel 1 von 1800 g auf 700 g reduziert. Die Ausbeute an DCBS betrug 91,5 %. Unter Einbeziehung der in der Mutterlauge gelösten Menge an Produkt ergab sich eine Selektivität von 95,5 %.

**Beispiel 5**

Anstelle von Dicyclohexylamin wurde unter Beispiel 1 analogen Bedingungen Dibenzylamin umgesetzt. Man erhielt das Produkt N,N-Dibenzyl-benzthiazolylsulfenamid) mit 91 % der Theorie. Unter Einbeziehung der in der Mutterlauge gelösten Menge an Produkt ergab sich eine Selektivität von 95 %. Der Wirkstoffgehalt des isolierten Produktes lag laut HPLC bei >99 %.

**Beispiel 6**

Wie in Beispiel 1 arbeitend setzte man statt Dicyclohexylamin Diisopropylamin ein. Man erhielt N,N-Diisopropyl-benzthiazolyl-sulfenamid mit einer Ausbeute von 85 % und einer Reinheit von >99 % (HPLC). Unter Einbeziehung der in der Mutterlauge gelösten Menge an Produkt (10 % der theoretischen Gesamtmenge) ergab sich eine Selektivität von 95 %.

## Patentansprüche

1. Verfahren zur Herstellung von gegebenenfalls substituierten N,N-Dialkyl-, -Dicycloalkyl, -Diaryl- oder -Diarylalkylbenzthiazolyl-sulfenamiden durch Umsetzung von gegebenenfalls substituierten 2-Mercaptobenzthiazolen und/oder den entsprechenden Disulfiden mit N,N-Dialkyl-, -Dicycloalkyl-, -Diaryl- oder -Diarylalkylaminen bei erhöhter Temperatur, dadurch gekennzeichnet, daß man die Umsetzung in Gegenwart von Chlor oder in Gegenwart eines Chlor/Inertgasgemisches sowie in Gegenwart von wasserfreien $C_1$-$C_4$-Alkoholen durchführt, wobei die Chlorierungszeit 10 bis 150 Minuten, das Molverhältnis von gegebenenfalls substituiertem 2-Mercapto-benzthiazol zu Amin 1:3 bis 1:4 und das Molverhältnis von gegebenenfalls substituiertem Dibenzthiazolyldisulfid zu Amin 1:4 bis 1:6 beträgt und wobei 300 bis 1500 g Alkohol pro Äquivalent 2-Mercaptobenzthiazol und 1 bis 1,6 Mol Chlor pro Mol gegebenenfalls substituiertem 2-Mercapto-benzthiazol oder 1 bis 1,5 Mol Chlor pro Mol gegebenenfalls substituiertem Dibenzthiazolyldisulfid zugesetzt werden.

2. Verfahren nach Anspruch 1, dadurch gekennzeichnet, daß man die Umsetzung bei Temperaturen von 40 bis 80°C durchführt.

3. Verfahren nach Ansprüchen 1 und 2, dadurch gekennzeichnet, daß die Chlorierungszeit 10 bis 60 Minuten beträgt.

Europäisches Patentamt

# EUROPÄISCHER RECHERCHENBERICHT

Nummer der Anmeldung
EP 95 12 0539

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | US-A-4 252 942 (ALICOT MICHEL ET AL) 24.Februar 1981<br>* das ganze Dokument *<br>--- | 1-3 | C07D277/80 |
| Y | US-A-3 600 398 (SVARZ JERRY J ET AL) 17.August 1971<br>Siehe insbesondere die Beispiele<br>--- | 1-3 | |
| Y | US-A-3 144 652 (D'AMICO (ASS. MONSANTO COMPANY)) 11.August 1964<br>Siehe Spalte 2, Zeile 19 und Beispiele 1 und 2<br>--- | 1-3 | |
| D,Y | DATABASE WPI<br>Section Ch, Week 8445<br>Derwent Publications Ltd., London, GB;<br>Class B03, AN 84-279280<br>& JP-A-59 172 483 ( KAWAGUCHI CHEM IND KK) , 29.September 1984<br>* Zusammenfassung *<br>--- | 1-3 | |
| | | | RECHERCHIERTE SACHGEBIETE (Int.Cl.6) |
| A | J. ORG. CHEM.,<br>Bd. 14, Nr. 6, 1949<br>Seiten 921-934, XP 000566968<br>CARR ET AL. 'Thiazolesulfenamides'<br>* Seite 924 - Seite 925 *<br>----- | 1-3 | C07D |

Der vorliegende Recherchenbericht wurde für alle Patentansprüche erstellt

| Recherchenort | Abschlußdatum der Recherche | Prüfer |
|---|---|---|
| MÜNCHEN | 9.April 1996 | Steendijk, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)